# FASCICULE DE BREVET EUROPEEN

(11) **EP 1 561 429 B1**
(45) Date de publication et mention de la délivrance du brevet: **09.01.2008**
(21) Numéro de dépôt: 04100413.6
(22) Date de dépôt: 04.02.2004
(51) Int. Cl.: A61B 17/80, F16B 35/06, F16B 23/00

(54) **Elément d'assemblage comportant une tête déformable**
Verbindungselement mit elastischem Kopf
Fastener having a deformable head

(43) Date de publication de la demande: 10.08.2005
(73) Titulaire: Bone and Joint Research S.A., 3650 Kayl (LU)
(72) Inventeur: Troussel, Serge, 6000, Charleroi (BE); Poffijn, Bart, 9820, Merelbeke (BE); Munting, Evrard, 1390, Biez (BE); Michielsen, Jef, 2320, Hoogstraten (BE); Thiltges, Paul, 1020, Bruxelles (BE); Bigand, Dominique, 62600, Groffliers (FR); Goube, Michel, 62152, Neufchatel Hardelot (FR); Merlot, Sylvain, 62780, Stella-Plage (FR)
(74) Mandataire: Schmitz, Jean-Marie

(56) Documents cités:
- WO-A-93/21848
- US-A1- 2002 045 898
- US-A1- 2003 105 462
- US-B1- 6 261 291
- US-B1- 6 306 136

## Description

La présente invention porte sur une conformation particulière des têtes des éléments d'assemblage.

Un élément d'assemblage, qu'il soit un clou, une vis, une clavette, ou autre, présente en général trois parties : une première partie qui réalise la connexion qu'on appelle le corps, par exemple le filet d'une vis, une deuxième partie qui permet l'assemblage appelée tête, une troisième et dernière partie qui permet la manipulation. Cette dernière partie est souvent logée dans la tête de l'élément d'assemblage.
La manoeuvre consiste en général à introduire l'élément d'assemblage dans les éléments à assembler par vissage, translation ou percussion.
Un lamage ou trou spécifique, est réalisé dans une des pièces à assembler, trou dans lequel vient se loger la tête de l'élément d'assemblage.
Dans certains cas, en particulier lorsque l'assemblage est soumis à des vibrations ou des efforts répétés (fatigue), l'utilisateur exige que la tête de l'élément d'assemblage soit fixée et condamnée dans ledit trou.
Dans d'autre cas, l'utilisateur exige en plus que la tête soit noyée dans l'élément à fixer, sans qu'elle en dépasse, et qu'il soit possible de démonter simplement l'ensemble.
Par exemple, dans une liaison par éléments filetée, l'ensemble, bien que maintenu par une vis et un écrou, peut, à cause de vibrations mettant en mouvement la vis, prendre du jeu, voire se désassembler. La vis n'est plus maintenue en rotation et par une combinaison de vibrations appropriées, se désengage de l'écrou.

Ce problème a été résolu de différentes manières, soit en positionnant un frein sur le filet de la vis, soit en condamnant par déformation la tête de l'élément de fixation, soit en soudant ou collant les dits éléments.

Le document US-B1-6 306 136 décrit un élément d'assemblage suivant le préambule de la revendication 1. Plus particulièrement, le document US-B1-6 306 136 un élément d'assemblage comportant un corps et une tête, la dite tête étant fendue en plusieurs endroits selon une direction proche angulairement de l'axe longitudinal du dit élément d'assemblage, et laissant apparaître un système de pétales. Ceux-ci comportent chacun un épaulement et un système de couronnes.

Le document US 2003/105 462 décrit un élément d'assemblage de ce type laissant apparaître un système de pétales qui comportent sur leur face supérieure une ou plusieurs mortaises.

Cependant ces méthodes ne permettent plus le démontage et souvent ne sont pas applicables dans certaines utilisations (par exemple dans les vis à os).

Ainsi, il n'est pas possible avec les moyens actuels de noyer la tête d'un élément d'assemblage dans son logement, sans qu'elle n'en dépasse, sans qu'elle ne s'y bloque naturellement, c'est à dire sans adjonction d'élément supplémentaire et sans qu'il soit possible de l'extraire à nouveau sans le détruire. Il s'agit de trois conditions simultanées.

L'invention suivante permet de remédier à ces inconvénients. Ce but ainsi que d'autres sont atteints, suivant l'invention, par un élément d'assemblage avant les caractéristiques de la partie caractérisante de la revendication 1. En effet, la tête de l'élément d'assemblage présente une conformation particulière.

En premier lieu, elle est fendue en plusieurs endroits de manière à ce que ces fentes donnent une possibilité de déformation. Ces fentes génèrent un système de pétales.

En deuxième lieu, on pratique sur chaque pétale, un système de couronnes placées de part et d'autre d'une rainure, couronnes dont la forme particulière permet la préhension avec un tournevis approprié.

En dernier lieu, on réalise dans la partie centrale de la tête de l'élément d'assemblage et selon un axe longitudinal, c'est à dire au milieu des pétales, un trou dont le fond présente une forme particulière, telle que un six pans ou un héxalobé par exemple ; cette forme particulière permet l'entraînement et la transmission des efforts et des couples au corps de l'élément d'assemblage ; cette conformation permet d'éviter la mise en charge de ladite tête. Ce dernier point permet de dissocier les fonctions préhension et mise en place de l'élément d'assemblage.

Cette combinaison de fonctions représentées par, tête déformable, couronne de préhension, et application de l'effort de mise en place et d'entraînement sur le corps, permet de manipuler aisément et jusqu'au bout l'élément d'assemblage : Puisque la tête est fendue, et que le système de couronnes de préhension est en retrait par rapport au bord externe de la tête, on peut introduire à terme, c'est à dire jusqu'au bout, l'élément d'assemblage dans son logement, même si celui-ci présente des dimensions identiques ou inférieures à ladite tête.
Cette conformation permet d'une part de loger une tête au moins plus grande d'un élément d'assemblage dans un trou plus petit de l'élément à fixer, et d'autre part, par une conformation particulière des surfaces en présence, respectivement de la tête et du trou, de bloquer la dite tête dans ledit trou.
En outre comme l'effort est appliqué dans la zone du corps de la vis, c'est à dire au-delà de la tête, la couronne de préhension ne supporte pas d'autres efforts que ceux apportés par la déformation.

Selon une caractéristique de l'invention, la tête de l'élément d'assemblage étant fendue en plusieurs endroits selon une direction proche angulairement de l'axe longitudinal de l'élément d'assemblage, les pétales qui en résultent, présentent un système de couronnes.
Celles-ci sont adaptées à sa préhension et à sa déformation.
Des modes particuliers de réalisation des couronnes sont possibles :
- les couronnes présentent une face externe sous la forme d'une queue d'aronde.
- les couronnes comportent une collerette.

Selon une autre caractéristique de l'invention, la tête de l'élément d'assemblage étant fendue en plusieurs endroits selon une direction proche angulairement de l'axe longitudinal de l'élément d'assemblage, les pétales qui en résultent, présentent chacun une rainure qui sépare deux couronnes, l'une interne, l'autre externe.
L'une des couronnes constitue le moyen de préhension, en particulier la couronne interne.
Des modes particuliers de réalisation des couronnes sont possibles :
- les couronnes de préhension présentent une forme en queue d'aronde
- les couronnes de préhension présentent une collerette

Selon une caractéristique qui ne fait pas partie de l'invention, la tête de l'élément d'assemblage étant fendue en plusieurs endroits selon une direction proche angulairement de l'axe longitudinal de l'élément d'assemblage, les pétales qui en résultent, présentent chacun sur leur face supérieure une ou plusieurs mortaises.

les couronnes de préhension présentent sur leur face supérieure des mortaises.

Ces mortaises constituent une prise pour les tenons d'un outil de manipulation.

Selon une autre caractéristique qui ne fait pas partie de l'invention, la tête de l'élément d'assemblage étant fendue en plusieurs endroits selon une direction proche angulairement de l'axe longitudinal de l'élément d'assemblage, les pétales qui en résultent, présentent chacun un épaulement et un système de couronnes, lesquelles couronnes offrent sur leur face latérale une ou plusieurs mortaises.

Selon une variante, les mortaises sont disposées sur la face supérieure des couronnes.
Ces mortaises constituent une prise pour les tenons d'un outil de manipulation.

Les dessins annexés illustrent l'invention :

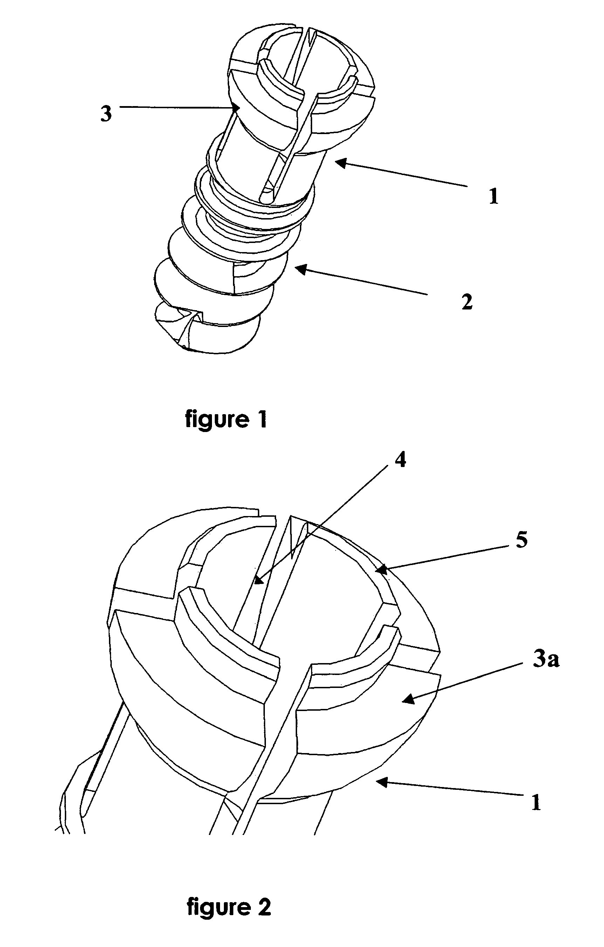

La figure 1 présente un élément d'assemblage tel une vis présentant un épaulement et un système de couronnes.
La figure 2 est une vue agrandie de la figure 1 en ce qui concerne sa tête.

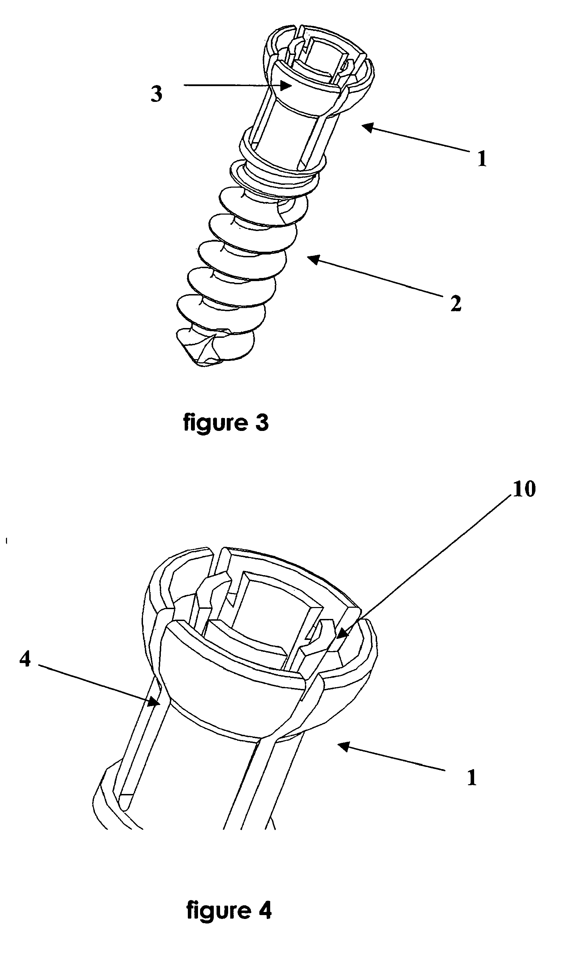

La figure 3 présente un élément d'assemblage tel une vis présentant deux systèmes de couronnes séparés par une rainure.
La figure 4 est une vue agrandie de la figure 3 en ce qui concerne sa tête.

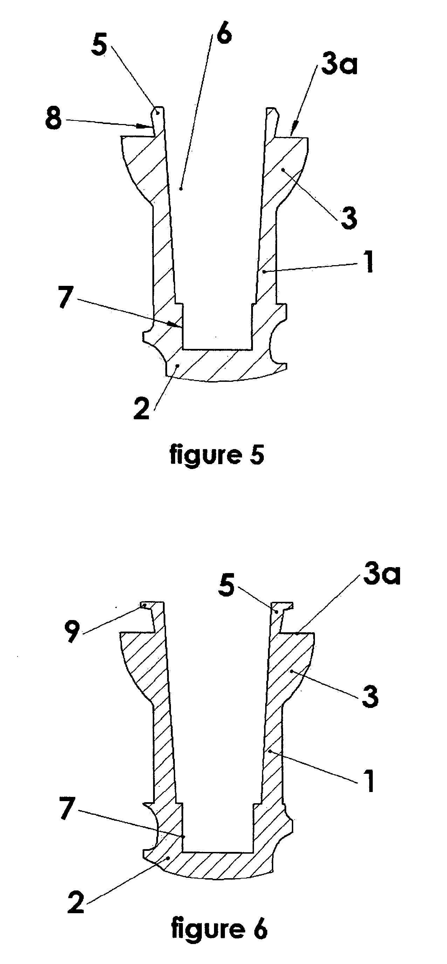

La figure 5 est une coupe longitudinale de la tête de la figure 2 où la couronne de préhension présente une queue d'aronde.
La figure 6 est une coupe longitudinale de la tête de la figure 2 où la couronne de préhension présente une collerette.

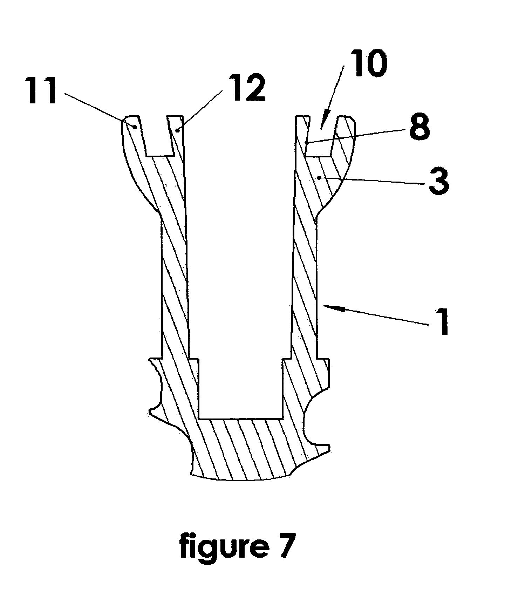

La figure 7 est une coupe longitudinale de la tête de la figure 4 où la couronne de préhension présente une queue d'aronde.
La figure 8 est une vue d'une vis avec son tournevis.
La figure 9 est une vue de détail agrandie de la connexion du tournevis sur la vis.
La figure 10 présente un élément d'assemblage tel une vis présentant sur la face supérieure des pétales, un système de mortaises.
La figure 11 présente un élément d'assemblage tel une vis présentant un système de couronnes disposées sur la partie supérieure de la tête et comportant des mortaises.
En référence à ces dessins, la tête (1) de l'élément d'assemblage comporte des pétales (3) pouvant se déformer grâce aux rainures longitudinales (4) réalisées dans la tête (1). Chaque pétale (3) comporte un épaulement (3a) et une couronne (5). Un trou central (6) présente dans le corps (2) de l'élément d'assemblage, un entraînement femelle (7). La face externe des couronnes présente une surface en forme de queue d'aronde (8).

Cette conformation permet à un moyen de préhension de se positionner sur les pétales (3) en épousant les surfaces en queue d'aronde (8). En appliquant un effort radial centripète sur les pétales (3) par l'intermédiaire d'un moyen de préhension (14), les rainures longitudinales (4) voient leur largeur diminuer, et la tête (1) se déforme ; on peut ainsi introduire l'ensemble de la tête (1) d'un l'élément d'assemblage dans un lamage dont les dimensions transversales sont plus petites, tout en manipulant la progression du corps (2) dans le logement receveur par l'entraînement central (7).

Selon une variante, la face externe de la couronne (5) présente une collerette(9).

Selon l'invention, chaque pétale (3) comporte une rainure circonférentielle (10) ouverte vers le sommet de l'élément d'assemblage et limitée par deux systèmes de couronnes (11) et (12), l'une externe (11), l'autre interne (12). La couronne interne (12) présente sur sa face externe, une forme en queue d'aronde (8).
Selon une variante non illustrée, cette face externe en queue d'aronde (8) peut être surmontée d'une collerette identique à la forme (9).

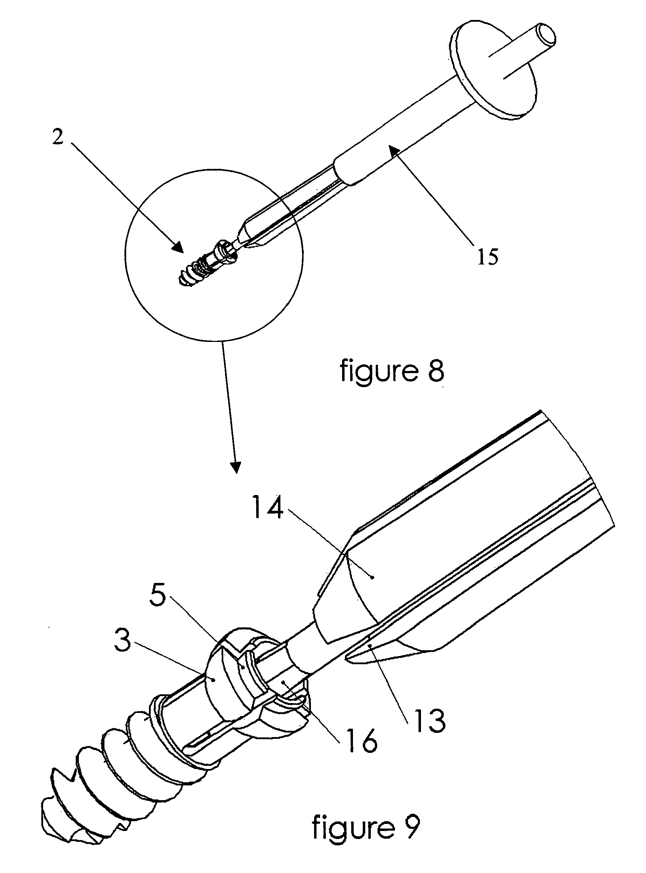

A titre d'exemple non limitatif, les figures 8 et 9 illustrent une autre variante comportant une vis à 4 pétales montée sur un tournevis approprié. Les lamelles (14) du tournevis viennent ceindre les couronnes (5) de la vis. Par déplacement de la coulisse (15) le long des lamelles (14), les pétales (3) se déforment par rétraction. En même temps, l'hexagone mâle (16) du tournevis entraîne la vis par la partie (7) du corps (2) de la vis. La déformation des pétales de la vis, permet d'introduire la tête de vis dans le lamage plus petit d'une plaque et s'y bloquer par déformation naturelle.

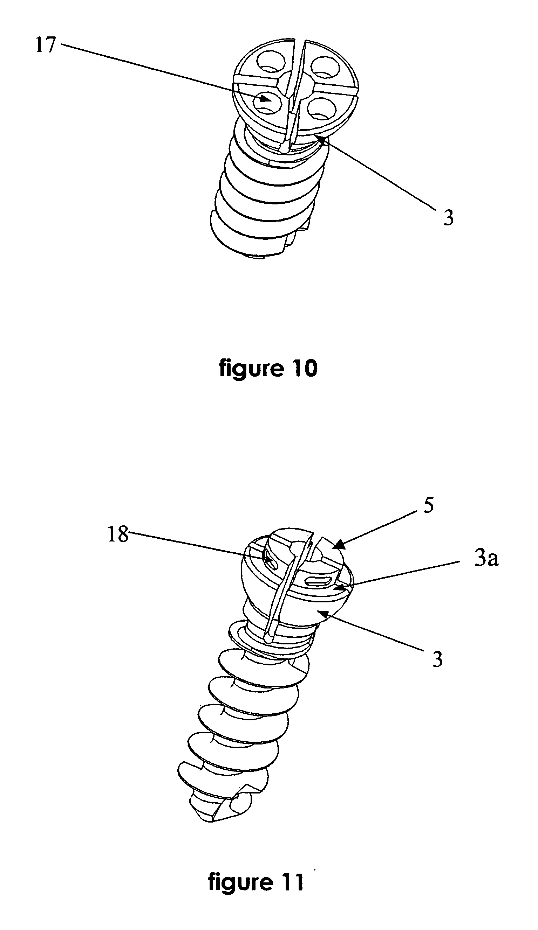

Les figures 10 et 11 illustrent une variante d'une vis qui comporte un système de mortaises (17) et (18) disposées respectivement sur la face supérieure (17) et sur la face latérale (18) de chaque pétale (3).

Ces éléments d'assemblage sont particulièrement utilisés comme vis dans les plaques orthopédiques.

## Revendications

1. Elément d'assemblage comportant un corps (2) et une tête (1), la dite tête (1) étant fendue en plusieurs endroits (4) selon une direction proche angulairement de l'axe longitudinal de l'élément d'assemblage, et laissant apparaître un système de pétales (3),
**caractérisé en ce que** les pétales (3) présentent chacun une rainure (10) qui sépare deux couronnes (11, 12), l'une interne (12), l'autre externe (11).

2. Elément d'assemblage selon la revendication 1, **caractérisé en ce que** les couronnes (12) présentent une face externe sous la forme d'une queue d'aronde (8).

3. Elément d'assemblage selon la revendication 1, **caractérisé en ce que** les couronnes (12) comportent une collerette (9).

## Claims

1. A fastener comprising a body (2) and a head (1), said head (1) being split in several locations (4) along a direction that is almost angular from the longitudinal axis of the fastener, and allowing a system of petals (3) to appear,
**Characterized in that** each of the petals (3) presents a groove (10) that separates two crowns (11, 12), one internal (12), the other external (11).

2. The fastener according to claim 1, **characterized in that** the crowns (12) present an external face in the form of a dovetail (8).

3. The fastener according to claim 1, **characterized in that** the crowns (12) comprise a flange (9).

## Patentansprüche

1. Zusammenbauelement, das einen Körper (2), einen Kopf (1) aufweist, wobei der Kopf (1) an mehreren Stellen (4) entlang einer Richtung, die winkelig der Längsachse des Zusammenbauelements nahe ist, gespaltet ist, und ein Keulensystem (3) erscheinen lässt,
**dadurch gekennzeichnet, dass** die Keulen (3) jeweils eine Rille (10) aufweisen, die zwei Kränze (11, 12), nämlich einen inneren (12) und einen äußeren (11) trennt.

2. Zusammenbauelement nach Anspruch 1, **dadurch gekennzeichnet, dass** die Kränze (12) eine Außenseite in Schwalbenschwanzform (8) aufweisen.

3. Zusammenbauelement nach Anspruch 1, **dadurch gekennzeichnet, dass** die Kränze (12) einen Kragen (9) aufweisen.
